# EUROPEAN PATENT APPLICATION

(11) **EP 3 088 002 A1**
(43) Date of publication of application: **02.11.2016**
(21) Application number: 15743275.8
(22) Date of filing: 30.01.2015
(51) Int. Cl.: A61K 47/02, A61K 9/16, A61K 9/20, A61K 47/38

(54) **EXTENDED RELEASE FORMULATION**

(30) Priority: 31.01.2014 JP 2014017333
(71) Applicant: Shionogi & Co., Ltd., Osaka-shi, Osaka 541-0045 (JP)
(72) Inventor: MIZUTANI, Naoya, Amagasaki-shi Hyogo 660-0813 (JP); KIMURA, Go, Amagasaki-shi Hyogo 660-0813 (JP); NISHINO, Yukiko, Amagasaki-shi Hyogo 660-0813 (JP); SAKUMA, Satoshi, Amagasaki-shi Hyogo 660-0813 (JP)
(74) Representative: Eder, Michael
(86) International application number: PCT/JP2015/052632
(87) International publication number: WO 2015/115586

(57) **Abstract**

The present invention is a sustained release formulation which is capable of cotrolling dissolution of a drug and of enduring peristaltic movement of the gastrointestinal tract. Specifically, the present invention is: a solid dosage form comprising a drug, croscarmellose sodium, an alkali metal salt or an alkoxide, and an alkali agent, wherein the alkali metal salt is a sodium salt or a potassium salt, and the alkali agent is a magnesium oxide; or a solid dosage form comprising a drug, croscarmellose sodium, an alkali metal salt or an alkoxide, and a water soluble polymer or a hydrophobic base, wherein the alkali metal salt is a sodium salt or a potassium salt. The present invention is thus capable of providing an extended release formulation that controls dissolution of a drug and endures peristaltic movement of the gastrointestinal tract.

## Description

### TECHNICAL FIELD

The present invention is a sustained release formulation comprising a drug, croscarmellose sodium, an alkali metal salt or an alkoxide, and an alkali agent, the alkali metal salt being a sodium salt or a potassium salt, and the alkali agent being a magnesium oxide. The present invention is also a sustained release formulation comprising a drug, croscarmellose sodium, an alkali metal salt or an alkoxide, and a water-soluble polymer or a hydrophobic base, the alkali metal salt being a sodium salt or a potassium salt.

### BACKGROUND ART

As a drug dissolve gradually in sustained release formulation, blood concentration of the drug can be constant for a prolonged time. Therefore, drug compliance can be better for the cause of reducing the number of drug administration time. There is a therapeutic range (treatment range) to indicate appropriate effect of a drug. When the blood concentration of the drug is becoming lower, an effect of the drug may be lost as much as the amount. On the contrary, when the blood concentration of the drug is becoming much higher, some adverse effect will be appeared. The blood concentration which the adverse effect has appeared is toxic range. The sustained release formulation may be able to reduce a risk to reach the blood concentration to the toxic range and reduce to the time of no effect range.

The sustained release formulation can control dissolution of a drug when, for example, the drug is mixed as matrix in the tablet. However, this formulation may be disintegrated by peristaltic movement of the gastrointestinal tract, and the drug may be dissolved quickly than planned time.

There are cases that croscarmellose sodium may be gelation when sodium ion of croscarmellose sodium is exchanged for sodium ion of sodium hydroxide or sodium carbonate under alkaline. By this gelation, a formulation containing a drug, croscarmellose sodium, sodium carbonate and calcium carbonate is disclosed in non-patent document 1. In this formulation, when croscarmellose sodium and alkali base such as sodium carbonate or calcium carbonate are increased, the dissolution rate may be decreased under time-dependent in conservation. However, the sustained release formulation isn't disclosed in the document.

Examples of formulation which is containing croscarmellose sodium and sodium salt include as follows references.

The formulation which is containing paracetamol as active ingredient, sodium bicarbonate and croscarmellose sodium is disclosed in patent document 1. However, this formulation dissolves paracetamol rapidly, and the sustained release formulation isn't disclosed in this document.

The formulation which contains sildenafil citrate as active ingredient, sodium bicarbonate and croscarmellose sodium is disclosed in patent document 2. However, this formulationdissolves sildenafil citrate rapidly, and the sustained release formulation isn't disclosed in this document.

### PRIOR ART REFERENCES

### [Patent Document]

[Patent Document 1] JP2008-500287
[Patent Document 2] JP2009-517346

### [Non-patent Document]

[Non-patent document 1] Pharmaceutical Development and Technology 2014; 19(3): 285-289

### SUMMARY OF THE INVENTION

### PROBLEMS TO BE SOLVED BY THE INVENTION

Therefore, the sustained release formulation was desired that the formulation is hardly disintegrated by peristaltic movement of the gastrointestinal tract and the drug is dissolved gradually from formulation in small intestine, duodenum, or large intestine.

### MEANS FOR SOLVING THE PROBLEM

The inventors of the present invention intensively studied and found the optimum formulation to develop the sustained release formulation hardly disintegrated by peristaltic movement of the gastrointestinal tract and the drug is dissolved gradually from formulation. Specifically, a solid dosage form comprising a drug, croscarmellose sodium, an alkali metal salt or an alkoxide, and an alkali agent, wherein the alkali metal salt is a sodium salt or a potassium salt, and the alkali agent is a magnesium oxide. Also, a solid dosage form comprising a drug, croscarmellose sodium, an alkali metal salt or an alkoxide, and a water soluble polymer or a hydrophobic base, wherein the alkali metal salt is a sodium salt or a potassium salt.

That is, the present invention relates to the following items:
(1) A solid dosage form comprising a drug, croscarmellose sodium, an alkali metal salt or an alkoxide, and an alkali agent, wherein the alkali metal salt is a sodium salt or a potassium salt, and the alkali agent is a magnesium oxide,
(2) The solid dosage form according to (1), which comprises the alkali metal salt, wherein the alkali metal salt is one or more selected from the group consisting of sodium carbonate, sodium hydrogen carbonate and potassium carbonate,
(3) The solid dosage form according to (2), wherein the alkali metal salt is sodium carbonate,
(4) A solid dosage form comprising a drug, croscarmellose sodium, an alkali metal salt or an alkoxide, and a water soluble polymer or a hydrophobic base, wherein the alkali metal salt is a sodium salt or a potassium salt,
(5) The solid dosage form according to (4), wherein the drug is a basic drug,
(6) The solid dosage form according to (4) or (5), which further comprises an alkali agent,
(7) The solid dosage form according to any one of the above items (4) to (6), which comprises an alkali metal salt, wherein the alkali metal salt is one or more selected from the group consisting of sodium carbonate, sodium hydrogen carbonate and potassium carbonate,
(8) The solid dosage form according to (6), wherein the alkali agent is one or more selected from the group consisting of magnesium oxide, magnesium hydroxide, aluminum magnesium hydroxide, synthetic hydrotalcite, calcium carbonate, magnesium carbonate, magnesium aluminometasilicate and calcium silicate,
(9) The solid dosage form according to (8), wherein the alkali agent is magnesium oxide,
(10) The solid dosage form according to any one of the above items (4) to (9), which comprises a hydrophobic base, wherein the hydrophobic base is a water-soluble polymer or oleaginous base,
(11) The solid dosage form according to (10), wherein the hydrophobic base is a water-soluble polymer, wherein the water-soluble polymer is one or more selected from the group consisting of cellulose-based polymer, vinyl-based polymer and acrylic-based polymer,
(12) The solid dosage form according to (11), wherein the water-soluble polymer is one or more selected from the group consisting of ethyl cellulose, low substituted hydroxypropyl cellulose, aminoalkylmetaacrylatecopolymer, polyethylacrylate-methylmethacrylate-trimethylammmonioethylmethacrylatechlorid, cellulose acetate phthalate, methacrylate copolymer, hydroxypropylmethylcellulose phthalate, hydroxypropylmethylcellulose acetate succinate, carboxymethylethyl cellulose and carboxyvinyl polymer,
(13) The solid dosage form according to (10), wherein the hydrophobic base is oleaginous base, wherein the oleaginous base is one or more selected from the group consisting of paraffin, microcrystalline wax, ceresin, vegetable wax, cacao butter, carnauba wax, beeswax, cetanol, stearyl alcohol, myristic acid, palmitic acid, stearic acid, glycerol fatty acid ester, polyglycerol fatty acid ester, glycerol free fatty acid ester, propylene glycol fatty acid ester, sorbitan fatty acid ester, sucrose fatty acid ester and hardened oil,
(14) The solid dosage form according to any one of the above items (4) to (9), which comprises a water-soluble polymer, wherein the water-soluble polymer is one or more selected from the group consisting of cellulose-based polymer, vinyl-based polymer and acrylic-based polymer,
(15) The solid dosage form according to (14), wherein the water-soluble polymer is one or more selected from the group consisting of hypromellose, crospovidone, povidone, sodium polyacrylate, sodium alginate and polyethylene oxide,
(16) The solid dosage form according to (6), which comprises an alkali metal salt, a water soluble polymer and an alkali agent, wherein the water soluble polymer is hypromellose and the alkali agent is magnesium oxide,
(17) The solid dosage form according to any one of the above items (1) to (16), wherein the content of the drug in the formulation is 0.01 to 90 %(w/w),
(18) The solid dosage form according to any one of the above items (1) to (3) and (6) to (17), wherein the content of the alkali agent in the formulation is 0.1 to 30 %(w/w),
(19) The solid dosage form according to (15) or (16), wherein the content of the hypromellose in the formulation is 0.1 to 30 %(w/w),
(20) The solid dosage form according to (3), wherein the content of the drug in the formulation is 0.01 to 90 %(w/w), and the content of magnesium oxide in the formulation is 0.1 to 30 %(w/w),
(21) The solid dosage form according to (16), wherein the content of the drug in the formulation is 0.01 to 90 %(w/w), the content of magnesium oxide in the formulation is 0.1 to 30 %(w/w), and the content of hypromellose in the formulation is 0.1 to 30 %(w/w),
(22) The solid dosage form according to any one of the above items (1) to (21), wherein the solid dosage form is a tablet or a granule.

### EFFECT OF THE INVENTION

The sustained release formulation of the present invention (hereinafter referred to "the present formulation") is sustainable form in peristaltic movement of the gastrointestinal tract along with controlling the dissolution rate of the drug.

### BRIEF DESCRIPTION OF THE DRAWINGS

Fig. 1 shows the dissolution behavior of the comparative example 1 and 2 (test solution: 2nd fluid for dissolution test).
Fig. 2 shows the dissolution behavior of the comparative example 1 and 2 (test solution: distilled water).
Fig. 3 shows the dissolution behavior of the comparative example 1 and 2 (test solution: 1st fluid for dissolution test).
Fig. 4 shows the dissolution behavior of the example 1 and the comparative 1 (test solution: 2nd fluid for dissolution test).
Fig. 5 shows the dissolution behavior of the comparative example 1 and the comparative 1 (test solution: distilled water).
Fig. 6 shows the dissolution behavior of the example 1 and the comparative 1 (test solution: 1 st fluid for dissolution test).
Fig. 7 shows the dissolution behavior of the example 1 to 4 and the comparative 1 (test solution: 2nd fluid for dissolution test).
Fig. 8 shows the dissolution behavior of the example 5 to 7 and the comparative 3 (test solution: 2nd fluid for dissolution test).
Fig. 9 shows the dissolution behavior of the example 8 to 11 and the comparative 4 (test solution: 2nd fluid for dissolution test).
Fig. 10 shows the dissolution behavior of the example 12 and 13, and the comparative 1 (test solution: 2nd fluid for dissolution test).

### EMBODIMENTS FOR CARRYING OUT THE INVENTION

In this description, various drugs can be used as a drug, and any type can be used such as solid, powder, crystal, oily, solution. Acid drug, neutral drug and basic drug can be used. As the drug used in the present invention, one or two or more kinds of components selected from a nutritional health supplement, an antipyretic-analgesic-antiphlogistic, a psychotropic, an anti-anxiety agent, an anti-depressant, a hypnotic-sedative, an antispasmodic, a central nerve-acting drug, a brain metabolism improving agent, a brain circulation improving agent, an antiepileptic, a sympathomimetic agent, a medicine for digestive system, an antacid, an anti-ulcer agent, an antitussive-expectorant, an antiemetic, a respiratory promoter, a bronchodilator, a drug for allergy, a dental oral drug, an anti-histamine agent, a cardiotonic agent, a drug for arrhythmia, a diuretic, an antihypertensive, a vasoconstrictor, a coronary vasodilator, a peripheral vasodilator, a drug for hyperlipemia, a cholagogue, an antibiotic, a chemotherapeutic, a drug for diabetes, a drug for osteoporosis, an antirheumatic, a skeletal muscle relaxant, an antispasmodic, a hormone agent, an alkaloid narcotic, a sulfa drug, a gout treating drug, a blood coagulation preventing agent, an anti-malignant tumor agent, and Alzheimer's disease treating drug are used.

A basic drug is the drug indicates the value more than pH 7 of solution pH when the drug is dissolved in water.

Examples of the nutritional health supplement include vitamins such as vitamin A, vitamin D, vitamin E (d-α-tocopherol acetate etc.), vitamin B1 (dibenzoylthiamine, fursultiamine hydrochloride etc.), vitamin B2 (riboflavine butyrate etc.), vitamin B6 (pyridoxine hydrochloride etc.), vitamin C (ascorbic acid, sodium L-ascorbate etc.), and vitamin B12 (hydroxocobalamine acetate, cyanocobalamine etc.), minerals such as calcium, magnesium and iron, proteins, amino-acids, oligosaccharides, and crude drugs.

Examples of the antipyretic-analgesic-antiphlogistic include aspirin, acetoaminophen, ethenzamide, ibuprofen, diphenhydramine hydrochloride, chlorpheniramine dl-maleate, dihydrocodeine phosphate, noscapine, methylephedrine hydrochloride, phenylpropanolamine hydrochloride, caffeine, anhydrous caffeine, serrapeptase, lysozyme chloride, tolfenamic acid, mefenamic acid, diclofenac sodium, flufenamic acid, salicylamide, aminopyrine, ketoprophen, indometacin, bucolome, and pentazocine. Examples of the psychtropic include chlorpromazine, and reserpine. Examples of the anti-anxiety drug include alprazolam, chlordiazepoxide, and diazepam. Examples of the anti-depressant include imipramine, maprotiline hydrochloride, and amphetamine. Examples of the hypnotic-sedative include estazolam, nitrazepam, diaaepam, perlapine, and phenobarbital sodium. Examples of the antispasmodic include scopolamine hydrobromide, diphenhydramine hydrochloride, and papaverine hydrochloride. Examples of the central nerve-acting drug include citicoline. Examples of the brain metabolism improving agent include meclofenoxate hydrochloride. Examples of the brain circulation improving agent include vinpocetine. Examples of the antiepileptic include phenytoin and carbamazepine. Examples of the sympathomimetic agent include isoproterenol hydrochloride.

Examples of the medicine for digestive system include stomachic digestants such as diastase, sugar-containing pepsin, scopolia extract, cellulase AP3, lipase AP, and cinnamon oil, and medicine for intestinal disorders such as berberine chloride, resistant lactic acid bacterium, and Bifidobacteria. Examples of the antacid include magnesium carbonate, sodium bicarbonate, magnesium aluminate metasilicate, synthetic hydrotalcite, precipitated calcium carbonate, and magnesium oxide. Examples of the antiulcer agent include lansoprazole, omeprazole, rabeprazole, famotidine, cimetidine, and ranitidine hydrochloride.

Examples of the antitussive-expectorant include cloperastine hydrochloride, dextromethorphan hydrobromide, theophylline, potassium guacacolsulfonate, guaifenesin, and codeine phosphate. Examples of the antiemetic include difenidol hydrochloride, and metoclopramide. Examples of the respiratory promoter include levallorphan tartrate. Examples of the bronchodilator include theophylline, and salbutamol sulfate. Examples of the drug for the allergy include amlexanox, and seratrodast.

Examples of the dental oral drug include oxytetracycline, triamcinolone acetomide, chlorhexidine hydrochloride, and lidocaine. Examples of the anti-histamine agent include diphenhydramine hydrochloride, promethazine, isothipendyl hydrochloride, and chlorpheniramine dl-maleate.

Examples of the cardiotonic agent include caffeine, and digoxin. Examples of the agent for arrhythmia include procaineamide hydrochloride, propranolol hydrochloride, and pindolol. Examples of the diuretic include isosorbide, furosemide, and thiazide agent such as HCTZ.

Examples of the antihypertensive include nicardipine, delapril hydrochloride, captopril, hexamethonium bromide, hydralazine hydrochloride, labetalol hydrochloride, manidipine hydrochloride, candesartan cilexetil, methyldopa, losartan, valsartan, eposartan, irbesartan, tasosartan, and telmisartan. Examples of the vasoconstrictor include phenylephrine hydrochloride. Examples of the coronary vasodilator include carbocromen hydrochloride, molsidomine, and verapamil hydrochloride. Examples of the peripheral vasodilator include cinnarizine. Examples of the agent for hyperlipemia include cerivastatin sodium, simvastatin, and pravastatin sodium. Examples of the cholagogue include dehydrocholic acid, and trepibutone.

Examples of the antibiotic include cephem antibiotics such as cephalexin, cefaclor, amoxicillin, pivmecillinam hydrochloride, cefotiam hexetil hydrochloride, cefadroxil, cefixime, cefditoren pivoxil, cefteram pivoxil, and cefpodoxymiproxetil, cefotiam hydrochloride, cefozopran hydrochloride, cefmenoxime hydrochloride, cefsulodin sodium, monobactam, penem and carbapenem antibiotics such as ampicillin, ciclacillin, sulbenicillin sodium, nalidixic acid, and synthetic anti-fungal agents such as enoxacin, and carumonam sodium.

Examples of the chemotherapeutic include sufamethizol, sulfamethizole hydrochloride, and thiazolsulfone. Examples of the agent for diabetes include tolbutamide, voglibose, pioglitazone hydrochloride, glibenclamide, troglitazon, rosiglitazone maleate, acarbose, miglitol, and emiglitate.

Examples of the agent for osteoporosis include ipriflavone. Examples of the skeletal muscle relaxant include methocarbamol. Examples of the antispasmodic include meclizine hydrochloride, and dimenhydrinate. Examples of the antirheumatic include methotrexate, and bucillamine. Examples of the hormone agent include liothyronine sodium, dexamethasone phosphate sodium, prednisolone, and oxendolone.

Examples of the alkaloid narcotic include opium, morphine hydrochloride, ipecac, oxycodone hydrochloride, opium alkaloid hydrochloride, and cocaine hydrochloride. Examples of the sulfa drug include sulfisomidine, and sufamethizol. Examples of the gout treating drug include allopurinol, and colchicine. Examples of the blood coagulation preventing agent include dicumarol. Examples of the anti-malignant tumor agent include 5-fluorouracil, uracil, and mitomycin. Examples of the Alzheimer's disease treating drug include donepezil hydrochloride, idebenone, and vinpocetine.

A content of drug in the present formulation may be a content at which drug efficacy can be arisen. For example, relative to the amount of the whole formulation, the content of drug is 0.01 to 90 %(w/w), preferably 0.025 to 80 %(w/w), more preferably 0.05 to 70 %(w/w). When the content of drug is larger than this content, there is a possibility that dissolution rate may not be able to decrease. When the content of drug is smaller than this content, there is a possibility that the drug efficacy cannot be arisen sufficiently.

In this description, croscarmellose sodium, those described in Japanese Pharmacopoeia, Japanese Pharmaceutical Codex, Japanese Pharmaceutical Excipients and Japanese Standard of Food Additives may be used. Croscarmellose sodium is crosslinked polymer of Carmellose sodium.

A content of croscarmellose sodium in the present formulation may be a certain amount of range at which drug can control dissolution rate of drug. Relative to the amount of the whole formulation, for example, the content of croscarmellose sodium is 1 to 30 %(w/w), preferably 2 to 25 %(w/w), more preferably 3 to 20 %(w/w). When the content of drug is larger or smaller than this content, there is a possibility that the formulation may be disintegrated immediately and that the formulation can't control drug dissolution.

In the present formulation, disintegrating agents can be used with croscarmellose sodium. Examples of combinable disintegrating agent include carmellose calcium, sodium starch glycolate, low substituted hydroxypropylcellulose and the like.

In this description, an alkali metal salt includes a salt similar intension with carboxylic acid or more weak acid than carboxylic acid. Examples of the alkali metal salt include sodium salt or potassium salt added carboxylic acid, carbonic acid or phenol acid.

In this description, sodium salt or potassium salt, those described in Japanese Pharmacopoeia, Japanese Pharmaceutical Codex, Japanese Pharmaceutical Excipients and Japanese Standard of Food Additives may be used. Croscarmellose sodium may be gelling by adding sodium salt or potassium salt into the present formulation, and dissolution rate of drug may be decelerated, and the formulation may be sustained release formulation. Examples of sodium salt or potassium salt include sodium hydroxide, sodium carbonate, sodium hydrogen carbonate, potassium hydroxide, potassium carbonate and the like. Sodium salt or potassium salt are preferably sodium carbonate or potassium carbonate.

A content of sodium salt or potassium salt in the present formulation may be a certain amount of range at which drug can control drug dissolution. For example, relative to the croscarmellose sodium 100 weight part, the content of sodium salt or potassium salt is 10 to 100 weight part, preferably 20 to 90 weight part, more preferably 30 to 80 weight part. Relative to the amount of the whole formulation, for example, the content of sodium salt or potassium salt is 0.1 to 30 %(w/w), preferably 0.5 to 25 %(w/w), more preferably 1 to 20 %(w/w). When the content of drug is larger or smaller than this content, there is a possibility that the formulation may be disintegrated immediately and that the formulation can't control drug dissolution.

In this description, alkoxide, those described in Japanese Pharmacopoeia, Japanese Pharmaceutical Codex, Japanese Pharmaceutical Excipients and Japanese Standard of Food Additives may be used. Alkoxide is a compound which has hydrogen atom of hydroxy group of alcohol substituted with metal. Examples of water-soluble polymer include Sodium methoxide and the like.

A content of alkoxide in the present formulation may be a certain amount of range at which drug can control drug dissolution. For example, relative to the croscarmellose sodium 100 weight part, the content of alkoxide is 10 to 100 weight part, preferably 20 to 90 weight part, more preferably 30 to 80 weight part. Relative to the amount of the whole formulation, for example, the content of sodium salt or potassium salt is 0.1 to 30 %(w/w), preferably 0.5 to 25 %(w/w), more preferably 1 to 20 %(w/w). When the content of drug is larger or smaller than this content, there is a possibility that the formulation may be disintegrated immediately and that the formulation can't control drug dissolution.

In this description, an alkali agent is not particularly limited as long as the pH of its 5 %(w/w) aqueous solution or a suspension is preferably 7 or more, and it may be a mixture of two or more types. Moreover, one that has a small dissolution rate in water is preferable. Examples include ones containing in the chemical formula one or more metal atom(s) selected from the group consisting of magnesium, calcium and aluminum. Preferably, the examples include one or more compounds selected from the group consisting of magnesium oxide, magnesium hydroxide, hydroxylation alumina magnesium, synthetic hydrotalcite, calcium carbonate, magnesium carbonate, magnesium aluminometa silicate, or calcium silicate, and more preferably they include magnesium oxide, magnesium hydroxide or a mixture thereof.

A content of alkali agent in the present formulation may be a certain amount of range at which drug can control elution of drug. For example, relative to the croscarmellose sodium 100 weight part, the content of alkali agent is 5 to 300 weight part, preferably 10 to 250 weight part, more preferably 50 to 200 weight part. Relative to the amount of the whole formulation, for example, the content of sodium salt or potassium salt is 0.1 to 30 %(w/w), preferably 0.5 to 25 %(w/w), more preferably 1 to 20 %(w/w). When the content of drug is larger or smaller than this content, there is a possibility that the formulation may be disintegrated immediately and that the formulation can't control drug dissolution.

In this description, water-soluble polymer, those described in Japanese Pharmacopoeia, Japanese Pharmaceutical Codex, Japanese Pharmaceutical Excipients and Japanese Standard of Food Additives may be used. Adding water-soluble polymer into the present formulation may hardly disintegrate the present formulation by peristaltic movement of the gastrointestinal tract. The water-soluble polymer may be dissolved into water and swelled. Specifically, examples of water-soluble polymer include cellulose-based polymer, starch-based polymer, vinyl-based polymer, acrylic-based polymer, and polyether. More specifically, hypromellose, hydroxypropyl cellulose, carboxymethyl cellulose, gelatinized starch, crospovidone, povidone, sodium polyacrylate, sodium alginate, polyethylene oxide and the like, preferably hypromellose.

A content of water-soluble polymer in the present formulation may be a certain amount of range at which drug can control elution of drug. For example, relative to the croscarmellose sodium 100 weight part, the content of alkali agent is 5 to 300 weight part, preferably 10 to 250 weight part, more preferably 50 to 200 weight part. Relative to the amount of the whole formulation, for example, the content of sodium salt or potassium salt is 0.1 to 30 %(w/w), preferably 0.5 to 25 %(w/w), more preferably 1 to 20 %(w/w). When the content of drug is larger than this content, there is a possibility that the formulation may not dissolve. When the content of drug is smaller than this content, there is a possibility that the formulation may disintegrate by peristaltic movement.

In this description, hydrophobic base, those described in Japanese Pharmacopoeia, Japanese Pharmaceutical Codex, Japanese Pharmaceutical Excipients and Japanese Standard of Food Additives may be used. Adding hydrophobic base into the present formulation may hardly disintegrate the present formulation by peristaltic movement of the gastrointestinal tract. Insolubule water-soluble polymer or oleaginous base may be used as hydrophobic base. As water-soluble polymer, specifically, cellulose-based polymer, vinyl-based polymer, and acrylic-based polymer are exemplified. More specifically, ethyl cellulose, low substituted hydroxypropyl cellulose, aminoalkylmetaacrylatecopolymer, polyethylacrylate-methylmethacrylate-trimethylammmonioethylmethacrylatechlorid, cellulose acetate phthalate, methacrylate copolymer, hydroxypropylmethylcellulose phthalate, hydroxypropylmethylcellulose acetate succinate, carboxymethylethyl cellulose and carboxyvinyl polymer are exemplified, preferably water-soluble polymer is ethyl cellulose. As oleaginous base, specifically, wax, paraffin, microcrystalline wax, ceresin, vegetable wax, cacao butter, carnauba wax, beeswax, cetanol, stearyl alcohol, myristic acid, palmitic acid, stearic acid, glycerol fatty acid ester, polyoxyethylene, polyglycerol fatty acid ester, glycerol free fatty acid ester, propylene glycol fatty acid ester, sorbitan fatty acid ester, sucrose fatty acid ester and hardened oil are exemplified.

A content of hydrophobic base in the present formulation may be a certain amount of range at which drug can control elution of drug. For example, relative to the croscarmellose sodium 100 weight part, the content of alkali agent is 5 to 300 weight part, preferably 10 to 250 weight part, more preferably 50 to 200 weight part. Relative to the amount of the whole formulation, for example, the content of sodium salt or potassium salt is 0.1 to 30 %(w/w), preferably 0.5 to 25 %(w/w), more preferably 1 to 20 %(w/w). When the content of drug is larger than this content, there is a possibility that the formulation may not swell and elute. When the content of drug is smaller than this content, there is a possibility that the formulation may disintegrate by peristaltic movement.

The preparation may contain an excipient, those described in Japanese Pharmacopoeia, Japanese Pharmaceutical Codex, Japanese Pharmaceutical Excipients and Japanese Standard of Food Additives may be used. Examples of excipient include powdered hydrogenated maltose starch syrup, glucose, fructose, lactose, D-mannitol, erythritol, maltitol, trehalose, sorbitol, sucrose, saccharose, fructo-oligosaccharide, palatinose, maltose (maltose), hydrogenated maltose starch, powdered syrup, starch syrup, fructose, lactulose, hydrogenated lactose lactitol, honey sugar, D-sorbitol, xylitol, corn starch, potato starch, wheat starch, rice starch, microcrystalline cellulose, silicic anhydride, anhydrous calcium phosphate, precipitated calcium carbonate, calcium silicate and the like. Preferably is powdered hydrogenated maltose starch syrup.

A content of the excipient is, for example, 1 to 80% by weight, preferably from 2.5 to 75% by weight, more preferably from 5 to 70 % by weight per preparation. When the content is larger than these contents, there is a possibility that a preparation in itself may upsize or a taking a lot of tablets. When the content is smaller than this content, the tablet cannot be formed.

The present preparation may contain a binding agent, those described in Japanese Pharmacopoeia, Japanese Pharmaceutical Codex, Japanese Pharmaceutical Excipients and Japanese Standard of Food Additives may be used. Examples of binding agent include hydroxypropylcellulose, corn starch, pregelatinized starch, partly pregelatinized starch, acacia, powdered acacia, gelatin, agar, dextrin, pullulan, polyvinylpyrrolidone, polyvinyl alcohol, microcrystalline cellulose, methylcellulose, ethylcellulose, carboxymethylethylcellulose, carmellose, carmellose sodium, hydroxyethyl cellulose, hydroxyethylmethylcellulose, hydroxypropylcellulose, hypromellose and the like. Preferably is hydroxypropylcellulose.

A content of the binding agent is, for example, 0.01 to 5% by weight, preferably from 0.05 to 4.5% by weight, more preferably from 0.1 to 4 % by weight per preparation. When the content is larger than these contents, there is a possibility that the yield of formulation may be decreased by adhesion to the manufacture instrument due to increased adhesiveness of formulation. When the content is smaller than this content, the formulation cannot be formed.

The present preparation may contain a lubricant, those described in Japanese Pharmacopoeia, Japanese Pharmaceutical Codex, Japanese Pharmaceutical Excipients and Japanese Standard of Food Additives may be used. Examples of lubricant include sucrose fatty acid ester, magnesium stearate (Taihei Chemical Industrial, NOF CORPORATION, SAKAI CHEMICAL INDUSTRY), calcium stearate, talc, hydrated silicon dioxide.

A content of the lubricant is, for example, 0.05 to 10 % by weight, preferably 0.075 to 7.5% by weight, more preferably 0.1 to 5 % by weight per preparation. When the content is larger than this content, there is a possibility that hardness of tablet or disintegration of tablet become low. When the content is smaller than this content, there is a possibility that a tablet cannot be formed.

The present preparation may contain an additive agent except those mentioned above, if necessary, those described in Japanese Pharmacopoeia, Japanese Pharmaceutical Codex, Japanese Pharmaceutical Excipients and Japanese Standard of Food Additives may be used. Moreover, the content of these additive agents may be a certain ratio. Examples of additive agent except those mentioned above include the flavor, glidant, color agent, taste masking agent coating agent, and the like.

Flavor includes flavoring agent. Examples of flavoring agent include banana flavor, sunfixbanana, orange extract, orange oil, caramel, camphor, cinnamon bark oil, spearmint oil, strawberry extract, chocolate extract, cherry flavor, sour orange oil, pine oil, mentha oil, vanilla flavor, bitter extract, fruit flavor, peppermint extract, mixture flavor, mint flavor, menthol, lemon powder, lemon oil, rose oil and the like.

Examples of glidant include hydrated silicon dioxide, light anhydrous silicic acid, microcrystalline cellulose, synthetic aluminum silicate, talc and the like. Preferably, fluidity agent is hydrated silicon dioxide.

Examples of color agent include yellow ferric oxide, red ferric oxide, Food red No.3, Food yellow No.5, Food blue No.1, brown oxide of iron, black oxide of iron, copper chlorophyll, copper chlorophyllin sodium, riboflavin, riboflavin butyrate, a green leaf extract end and the like.

Examples of taste masking agent include aspartame, sucralose, glycine, sodium chloride, magnesium chloride, hydrochloric acid, dilute hydrochloric acid, citric acid and the salt (sodium citrate), anhydrous citric acid, L-glutaminic acid and the salt, succinic acid and the salt, acetic acid, tartaric acid and the salt, sodium hydrogen carbonate, fumaric acid and the salt, malic acid and the salt, glacial acetic acid, inosinic acid disodium, honey and the like.

Examples of a coating agent include polyvinyl alcohol, ethyl cellulose, carboxymethylethylcellulose, carmellose, carmellose sodium, hydroxyethylcellulose, hydroxyethylmethylcellulose, hydroxypropylcellulose, hydroxypropyl methylcellulose, PVA copolymer, acrylic acid ethyl-methyl methacrylate copolymer dispersion liquid, amino alkyl methacrylate copolymer, opadry, carnabaro, carboxy vinyl polymers, dry methacrylate copolymer, dimethylamino ethyl methacrylate-methyl methacrylate copolymer, stearyl alcohol, shellac, cetanol, hydroxypropylmethylcellulose acetate succinate, hydroxypropylmethylcellulose phthalate, fumaric acid-stearic acid-polyvinyl acetal diethylamino acetate-hydroxypropyl methylcellulose mixture, polyvinyl acetal diethylamino acetate, polyvinyl alcohol, methacrylate copolymer, 2-methyl-5-vinyl pyridine methyl acrylate-methacrylic acid copolymer and the like.

The form of the present formulation may be a solid, generally, tablet or granule as described in the General Rules for Preparations in the Japanese Pharmacopoeia

A manufacturing method of the present formulation is 1) a drug and additive agent(s) are mixed, and the formulation is manufactured from the mixture by tablet compression "direct powder compression method", 2) a drug and additive agent(s) are mixed and granulated, further the formulation is manufactured from the granulated substance by tablet compression "granule compression method", 3) additive agent(s) are mixed and granulated, after that the granulated substance and a drug are mixed, and the formulation is manufactured by tablet compression. The preferable method of manufacture is the granule compression method. In 2) case, the granulated substance may be manufactured by granulating method normally using in pharmaceutically applicable. Extruding granulation method, agitation granulation method, fluidized bed granulation method, oscillating granulation method and the like are exemplified. Further, the granulated substance may be covered with coating agent.

Tablet compression may be conducted with a device for conducting external lubrication tablet compression, a single punch tablet machine, a rotary tablet machine and others.

The forming of the present formulation may be adopted as any shape, for example, round shape, elliptical shape, spherical shape, stick shape, torus shape, and lamination layer tablet, presscoated tablet. Further the formulation may be coated by coating. Some engraved mark(s) such as marks or characters, and cleavage lines may be applied for improvement of identification.

The diameter of the present formulation may be a moderate size for taking the drug.

Drug and additive agent in the present formulation may be used with a certain quantity by itself or mixture except when the formulation reveal an abnormality of dissolution of tablet or abnormality of disintegrating by peristaltic movement of the gastrointestinal tract. Preferable combination of drug and additive agent is, 1) drug/croscarmellose sodium/sodium salt/magnesium oxide, 2) drug/croscarmellose sodium/potassium salt/magnesium oxide, 3) drug/croscarmellose sodium/sodium salt/potassium salt/magnesium oxide, 4) drug/croscarmellose sodium/sodium salt/alkali agent/water-soluble polymer, 5) drug/croscarmellose sodium/potassium salt/alkali agent/water-soluble polymer, 6) drug/croscarmellose sodium/sodium salt/potassium salt/alkali agent/water-soluble polymer, 7) drug/croscarmellose sodium/sodium salt/water-soluble polymer, 8) drug/croscarmellose sodium/potassium salt/water-soluble polymer, 9) drug/croscarmellose sodium/sodium salt/potassium salt/water-soluble polymer, 10) drug/croscarmellose sodium/sodium salt/alkali agent/hydrophobic base, 11) drug/croscarmellose sodium/potassium salt/alkali agent/hydrophobic base, 12) drug/croscarmellose sodium/sodium salt/potassium salt/alkali agent/hydrophobic base, 13) drug/croscarmellose sodium/sodium salt/hydrophobic base, 14) drug/croscarmellose sodium/potassium salt/hydrophobic base, 15) drug/croscarmellose sodium/sodium salt/potassium salt/hydrophobic base, 16) drug/croscarmellose sodium/sodium carbonate/magnesium oxide/hypromellose, 17) drug/croscarmellose sodium/potassium carbonate/magnesium oxide/hypromellose, 18) drug/croscarmellose sodium/sodium carbonate/potassium carbonate/magnesium oxide/hypromellose, 19) drug/croscarmellose sodium/sodium carbonate/hypromellose, 20) drug/croscarmellose sodium/potassium carbonate/hypromellose, 21) drug/croscarmellose sodium/sodium carbonate/potassium carbonate/hypromellose, 22) drug/croscarmellose sodium/sodium carbonate/magnesium oxide/ethyl cellulose, 23) drug/croscarmellose sodium/potassium carbonate/magnesium oxide/ethyl cellulose, 24) drug/croscarmellose sodium/sodium carbonate/potassium carbonate/magnesium oxide/ethyl cellulose, 25) drug/croscarmellose sodium/sodium carbonate/ethyl cellulose, 26) drug/croscarmellose sodium/potassium carbonate/ethyl cellulose, and 27) drug/croscarmellose sodium/sodium carbonate/potassium carbonate/ethyl cellulose.

In a combination of drug/croscarmellose sodium/sodium salt/magnesium oxide, each content relative to the amount of the whole formulation is, for example, drug is 0.01 to 90 %(w/w), croscarmellose sodium is 1 to 30 %(w/w), sodium salt is 0.1 to 30 %(w/w), magnesium oxide is 0.1 to 30 %(w/w), preferably, drug is 0.025 to 80 %(w/w), croscarmellose sodium is 2 to 25 %(w/w), sodium salt is 0.5 to 25 %(w/w), magnesium oxide is 0.5 to 25 %(w/w), more preferably, drug is 0.05 to 70 %(w/w), croscarmellose sodium is 3 to 20 %(w/w), sodium salt is 1 to 20 %(w/w), magnesium oxide is 1 to 20 %(w/w).

In a combination of drug/croscarmellose sodium/potassium salt/magnesium oxide, each content relative to the amount of the whole formulation is, for example, drug is 0.01 to 90 %(w/w), croscarmellose sodium is 1 to 30 %(w/w), potassium salt is 0.1 to 30 %(w/w), magnesium oxide is 0.1 to 30 %(w/w), preferably, drug is 0.025 to 80 %(w/w), croscarmellose sodium is 2 to 25 %(w/w), potassium salt is 0.5 to 25 %(w/w), magnesium oxide is 0.5 to 25 %(w/w), more preferably, drug is 0.05 to 70 %(w/w), croscarmellose sodium is 3 to 20 %(w/w), potassium salt is 1 to 20 %(w/w), magnesium oxide is 1 to 20 %(w/w).

In a combination of drug/croscarmellose sodium/sodium salt/potassium salt/magnesium oxide, each content relative to the amount of the whole formulation is, for example, drug is 0.01 to 90 %(w/w), croscarmellose sodium is 1 to 30 %(w/w), sodium salt and potassium salt is 0.1 to 30 %(w/w), magnesium oxide is 0.1 to 30 %(w/w), preferably, drug is 0.025 to 80 %(w/w), croscarmellose sodium is 2 to 25 %(w/w), sodium salt and potassium salt is 0.5 to 25 %(w/w), magnesium oxide is 0.5 to 25 %(w/w), more preferably, drug is 0.05 to 70 %(w/w), croscarmellose sodium is 3 to 20 %(w/w), sodium salt and potassium salt is 1 to 20 %(w/w), magnesium oxide is 1 to 20 %(w/w).

In a combination of drug/croscarmellose sodium/sodium salt/alkali agent/water-soluble polymer, each content relative to the amount of the whole formulation is, for example, drug is 0.01 to 90 %(w/w), croscarmellose sodium is 1 to 30 %(w/w), sodium salt is 0.1 to 30 %(w/w), alkali agent is 0.1 to 30 %(w/w), water-soluble polymer is 0.1 to 30 %(w/w), preferably, drug is 0.025 to 80 %(w/w), croscarmellose sodium is 2 to 25 %(w/w), sodium salt is 0.5 to 25 %(w/w), alkali agent is 0.5 to 25 %(w/w), water-soluble polymer is 0.5 to 25 %(w/w), more preferably, drug is 0.05 to 70 %(w/w), croscarmellose sodium is 3 to 20 %(w/w), sodium salt is 1 to 20 %(w/w), alkali agent is 1 to 20 %(w/w), water-soluble polymer is 1 to 20 %(w/w).

In a combination of drug/croscarmellose sodium/potassium salt/alkali agent/water-soluble polymer, each content relative to the amount of the whole formulation is, for example, drug is 0.01 to 90 %(w/w), croscarmellose sodium is 1 to 30 %(w/w), potassium salt is 0.1 to 30 %(w/w), alkali agent is 0.1 to 30 %(w/w), water-soluble polymer is 0.1 to 30 %(w/w), preferably, drug is 0.025 to 80 %(w/w), croscarmellose sodium is 2 to 25 %(w/w), potassium salt is 0.5 to 25 %(w/w), alkali agent is 0.5 to 25 %(w/w), water-soluble polymer is 0.5 to 25 %(w/w), more preferably, drug is 0.05 to 70 %(w/w), croscarmellose sodium is 3 to 20 %(w/w), potassium salt is 1 to 20 %(w/w), alkali agent is 1 to 20 %(w/w), water-soluble polymer is 1 to 20 %(w/w).

In a combination of drug/croscarmellose sodium/sodium salt/potassium salt/alkali agent/water-soluble polymer, each content relative to the amount of the whole formulation is, for example, drug is 0.01 to 90 %(w/w), croscarmellose sodium is 1 to 30 %(w/w), sodium salt and potassium salt is 0.1 to 30 %(w/w), alkali agent is 0.1 to 30 %(w/w), water-soluble polymer is 0.1 to 30 %(w/w), preferably, drug is 0.02.5 to 80 %(w/w), croscarmellose sodium is 2 to 25 %(w/w), sodium salt and potassium salt is 0.5 to 25 %(w/w), alkali agent is 0.5 to 25 %(w/w), water-soluble polymer is 0.5 to 25 %(w/w), more preferably, drug is 0.05 to 70 %(w/w), croscarmellose sodium is 3 to 20 %(w/w), sodium salt and potassium salt is 1 to 20 %(w/w), alkali agent is 1 to 20 %(w/w), water-soluble polymer is 1 to 20 %(w/w).

In a combination of drug/croscarmellose sodium/sodium salt/water-soluble polymer, each content relative to the amount of the whole formulation is, for example, drug is 0.01 to 90 %(w/w), croscarmellose sodium is 1 to 30 %(w/w), sodium salt is 0.1 to 30 %(w/w), water-soluble polymer is 0.1 to 30 %(w/w), preferably, drug is 0.025 to 80 %(w/w), croscarmellose sodium is 2 to 25 %(w/w), sodium salt is 0.5 to 25 %(w/w), water-soluble polymer is 0.5 to 25 %(w/w), more preferably, drug is 0.05 to 70 %(w/w), croscarmellose sodium is 3 to 20 %(w/w), sodium salt is 1 to 20 %(w/w), water-soluble polymer is 1 to 20 %(w/w).

In a combination of drug/croscarmellose sodium/potassium salt/water-soluble polymer, each content relative to the amount of the whole formulation is, for example, drug is 0.01 to 90 %(w/w), croscarmellose sodium is 1 to 30 %(w/w), potassium salt is 0.1 to 30 %(w/w), water-soluble polymer is 0.1 to 30 %(w/w), preferably, drug is 0.25 to 80 %(w/w), croscarmellose sodium is 2 to 25 %(w/w), potassium salt is 0.5 to 25 %(w/w), water-soluble polymer is 0.5 to 25 %(w/w), more preferably, drug is 0.05 to 70 %(w/w), croscarmellose sodium is 3 to 20 %(w/w), potassium salt is 1 to 20 %(w/w), water-soluble polymer is 1 to 20 %(w/w).

In a combination of drug/croscarmellose sodium/sodium salt/potassium salt/water-soluble polymer, each content relative to the amount of the whole formulation is, for example, drug is 0.01 to 90 %(w/w), croscarmellose sodium is 1 to 30 %(w/w), sodium salt and potassium salt is 0.1 to 30 %(w/w), water-soluble polymer is 0.1 to 30 %(w/w), preferably, drug is 0.025 to 80 %(w/w), croscarmellose sodium is 2 to 25 %(w/w), sodium salt and potassium salt is 0.5 to 25 %(w/w), water-soluble polymer is 0.5 to 25 %(w/w), more preferably, drug is 0.05 to 70 %(w/w), croscarmellose sodium is 3 to 20 %(w/w), sodium salt and potassium salt is 1 to 20 %(w/w), water-soluble polymer is 1 to 20 %(w/w).

In a combination of drug/croscarmellose sodium/sodium salt/alkali agent/hydrophobic base, each content relative to the amount of the whole formulation is, for example, drug is 0.01 to 90 %(w/w), croscarmellose sodium is 1 to 30 %(w/w), sodium salt is 0.1 to 30 %(w/w), alkali agent is 0.1 to 30 %(w/w), hydrophobic base is 0.1 to 30 %(w/w), preferably, drug is 0.025 to 80 %(w/w), croscarmellose sodium is 2 to 25 %(w/w), sodium salt is 0.5 to 25 %(w/w), alkali agent is 0.5 to 25 %(w/w), hydrophobic base is 0.5 to 25 %(w/w), more preferably, drug is 0.05 to 70 %(w/w), croscarmellose sodium is 3 to 20 %(w/w), sodium salt is 1 to 20 %(w/w), alkali agent is 1 to 20 %(w/w), hydrophobic base is 1 to 20 %(w/w).

In a combination of drug/croscarmellose sodium/potassium salt/alkali agent/hydrophobic base, each content relative to the amount of the whole formulation is, for example, drug is 0.01 to 90 %(w/w), croscarmellose sodium is 1 to 30 %(w/w), potassium salt is 0.1 to 30 %(w/w), alkali agent is 0.1 to 30 %(w/w), hydrophobic base is 0.1 to 30 %(w/w), preferably, drug is 0.025 to 80 %(w/w), croscarmellose sodium is 2 to 25 %(w/w), potassium salt is 0.5 to 25 %(w/w), alkali agent is 0.5 to 25 %(w/w), hydrophobic base is 0.5 to 25 %(w/w), more preferably, drug is 0.05 to 70 %(w/w), croscarmellose sodium is 3 to 20 %(w/w), potassium salt is 1 to 20 %(w/w), alkali agent is 1 to 20 %(w/w), hydrophobic base is 1 to 20 %(w/w).

In a combination of drug/croscarmellose sodium/sodium salt/potassium salt/alkali agent/hydrophobic base, each content relative to the amount of the whole formulation is, for example, drug is 0.01 to 90 %(w/w), croscarmellose sodium is 1 to 30 %(w/w), sodium salt and potassium salt is 0.1 to 30 %(w/w), alkali agent is 0.1 to 30 %(w/w), hydrophobic base is 0.1 to 30 %(w/w), preferably, drug is 0.025 to 80 %(w/w), croscarmellose sodium is 2 to 25 %(w/w), sodium salt and potassium salt is 0.5 to 25 %(w/w), alkali agent is 0.5 to 25 %(w/w), hydrophobic base is 0.5 to 25 %(w/w), more preferably, drug is 0.05 to 70 %(w/w), croscarmellose sodium is 3 to 20 %(w/w), sodium salt and potassium salt is 1 to 20 %(w/w), alkali agent is 1 to 20 %(w/w), hydrophobic base is 1 to 20 %(w/w).

In a combination of drug/croscarmellose sodium/sodium salt/hydrophobic base, each content relative to the amount of the whole formulation is, for example, drug is 0.01 to 90 %(w/w), croscarmellose sodium is 1 to 30 %(w/w), sodium salt is 0.1 to 30 %(w/w), hydrophobic base is 0.1 to 30 %(w/w), preferably, drug is 0.025 to 80 %(w/w), croscarmellose sodium is 2 to 25 %(w/w), sodium salt is 0.5 to 25 %(w/w), hydrophobic base is 0.5 to 25 %(w/w), more preferably, drug is 0.05 to 70 %(w/w), croscarmellose sodium is 3 to 20 %(w/w), sodium salt is 1 to 20 %(w/w), hydrophobic base is 1 to 20 %(w/w).

In a combination of drug/croscarmellose sodium/potassium salt/hydrophobic base, each content relative to the amount of the whole formulation is, for example, drug is 0.01 to 90 %(w/w), croscarmellose sodium is 1 to 30 %(w/w), potassium salt is 0.1 to 30 %(w/w), hydrophobic base is 0.1 to 30 %(w/w), preferably, drug is 0.25 to 80 %(w/w), croscarmellose sodium is 2 to 25 %(w/w), potassium salt is 0.5 to 25 %(w/w), hydrophobic base is 0.5 to 25 %(w/w), more preferably, drug is 0.05 to 70 %(w/w), croscarmellose sodium is 3 to 20 %(w/w), potassium salt is 1 to 20 %(w/w), hydrophobic base is 1 to 20 %(w/w).

In a combination of drug/croscarmellose sodium/sodium salt/potassium salt/hydrophobic base, each content relative to the amount of the whole formulation is, for example, drug is 0.01 to 90 %(w/w), croscarmellose sodium is 1 to 30 %(w/w), sodium salt and potassium salt is 0.1 to 30 %(w/w), hydrophobic base is 0.1 to 30 %(w/w), preferably, drug is 0.025 to 80 %(w/w), croscarmellose sodium is 2 to 25 %(w/w), sodium salt and potassium salt is 0.5 to 25 %(w/w), hydrophobic base is 0.5 to 25 %(w/w), more preferably, drug is 0.05 to 70 %(w/w), croscarmellose sodium is 3 to 20 %(w/w), sodium salt and potassium salt is 1 to 20 %(w/w), hydrophobic base is 1 to 20 %(w/w).

In a combination of drug/croscarmellose sodium/sodium carbonate/magnesium oxide/hypromellose, each content relative to the amount of the whole formulation is, for example, drug is 0.01 to 90 %(w/w), croscarmellose sodium is 1 to 30 %(w/w), sodium carbonate is 0.1 to 30 %(w/w), magnesium oxide is 0.1 to 30 %(w/w), hypromellose is 0.1 to 30 %(w/w), preferably, drug is 0.025 to 80 %(w/w), croscarmellose sodium is 2 to 25 %(w/w), sodium carbonate is 0.5 to 25 %(w/w), magnesium oxide is 0.5 to 25 %(w/w), hypromellose is 0.5 to 25 %(w/w), more preferably, drug is 0.05 to 70 %(w/w), croscarmellose sodium is 3 to 20 %(w/w), sodium carbonate is 1 to 20 %(w/w), magnesium oxide is 1 to 20 %(w/w), hypromellose is 1 to 20 %(w/w).

In a combination of drug/croscarmellose sodium/potassium carbonate/magnesium oxide/hypromellose, each content relative to the amount of the whole formulation is, for example, drug is 0.01 to 90 %(w/w), croscarmellose sodium is 1 to 30 %(w/w), potassium carbonate is 0.1 to 30 %(w/w), magnesium oxide is 0.1 to 30 %(w/w), hypromellose is 0.1 to 30 %(w/w), preferably, drug is 0.025 to 80 %(w/w), croscarmellose sodium is 2 to 25 %(w/w), potassium carbonate is 0.5 to 25 %(w/w), magnesium oxide is 0.5 to 25 %(w/w), hypromellose is 0.5 to 25 %(w/w), more preferably, drug is 0.05 to 70 %(w/w), croscarmellose sodium is 3 to 20 %(w/w), potassium carbonate is 1 to 20 %(w/w), magnesium oxide is 1 to 20 %(w/w), hypromellose is 1 to 20 %(w/w).

In a combination of drug/croscarmellose sodium/sodium carbonate/potassium carbonate/magnesium oxide/hypromellose, each content relative to the amount of the whole formulation is, for example, drug is 0.01 to 90 %(w/w), croscarmellose sodium is 1 to 30 %(w/w), sodium carbonateand potassium carbonate is 0.1 to 30 %(w/w), magnesium oxide is 0.1 to 30 %(w/w), hypromellose is 0.1 to 30 %(w/w), preferably, drug is 0.025 to 80 %(w/w), croscarmellose sodium is 2 to 25 %(w/w), sodium carbonateand potassium carbonate is 0.5 to 25 %(w/w), magnesium oxide is 0.5 to 25 %(w/w), hypromellose is 0.5 to 25 %(w/w), more preferably, drug is 0.05 to 70 %(w/w), croscarmellose sodium is 3 to 20 %(w/w), sodium carbonateand potassium carbonate is 1 to 20 %(w/w), magnesium oxide is 1 to 20 %(w/w), hypromellose is 1 to 20 %(w/w).

In a combination of drug/croscarmellose sodium/sodium carbonate/hypromellose, each content relative to the amount of the whole formulation is, for example, drug is 0.01 to 90 %(w/w), croscarmellose sodium is 1 to 30 %(w/w), sodium carbonate is 0.1 to 30 %(w/w), hypromellose is 0.1 to 30 %(w/w), preferably, drug is 0.02.5 to 80 %(w/w), croscarmellose sodium is 2 to 25 %(w/w), sodium carbonate is 0.5 to 25 %(w/w), hypromellose is 0.5 to 25 %(w/w), more preferably, drug is 0.05 to 70 %(w/w), croscarmellose sodium is 3 to 20 %(w/w), sodium carbonate is 1 to 20 %(w/w), hypromellose is 1 to 20 %(w/w).

In a combination of drug/croscarmellose sodium/potassium carbonate/hypromellose, each content relative to the amount of the whole formulation is, for example, drug is 0.01 to 90 %(w/w), croscarmellose sodium is 1 to 30 %(w/w), potassium carbonate is 0.1 to 30 %(w/w), hypromellose is 0.1 to 30 %(w/w), preferably, drug is 0.025 to 80 %(w/w), croscarmellose sodium is 2 to 25 %(w/w), magnesium oxide is 0.5 to 25 %(w/w), hypromellose is 0.5 to 25 %(w/w), more preferably, drug is 0.05 to 70 %(w/w), croscarmellose sodium is 3 to 20 %(w/w), magnesium oxide is 1 to 20 %(w/w), hypromellose is 1 to 20 %(w/w).

In a combination of drug/croscarmellose sodium/sodium carbonate/potassium carbonate/hypromellose, each content relative to the amount of the whole formulation is, for example, drug is 0.01 to 90 %(w/w), croscarmellose sodium is 1 to 30 %(w/w), sodium carbonateand potassium carbonate is 0.1 to 30 %(w/w), hypromellose is 0.1 to 30 %(w/w), preferably, drug is 0.025 to 80 %(w/w), croscarmellose sodium is 2 to 25 %(w/w), sodium carbonateand potassium carbonate is 0.5 to 25 %(w/w), hypromellose is 0.5 to 25 %(w/w), more preferably, drug is 0.05 to 70 %(w/w), croscarmellose sodium is 3 to 20 %(w/w), sodium carbonateand potassium carbonate is 1 to 20 %(w/w), hypromellose is 1 to 20 %(w/w).

In a combination of drug/croscarmellose sodium/sodium carbonate/magnesium oxide/ethyl cellulose, each content relative to the amount of the whole formulation is, for example, drug is 0.01 to 90 %(w/w), croscarmellose sodium is 1 to 30 %(w/w), sodium carbonate is 0.1 to 30 %(w/w), magnesium oxide is 0.1 to 30 %(w/w), ethyl cellulose is 0.1 to 30 %(w/w), preferably, drug is 0.025 to 80 %(w/w), croscarmellose sodium is 2 to 25 %(w/w), sodium carbonate is 0.5 to 25 %(w/w), magnesium oxide is 0.5 to 25 %(w/w), ethyl cellulose is 0.5 to 25 %(w/w), more preferably, drug is 0.05 to 70 %(w/w), croscarmellose sodium is 3 to 20 %(w/w), sodium carbonate is 1 to 20 %(w/w), magnesium oxide is 1 to 20 %(w/w), ethyl cellulose is 1 to 20 %(w/w).

In a combination of drug/croscarmellose sodium/potassium carbonate/magnesium oxide/ethyl cellulose, each content relative to the amount of the whole formulation is, for example, drug is 0.01 to 90 %(w/w), croscarmellose sodium is 1 to 30 %(w/w), potassium carbonate is 0.1 to 30 %(w/w), magnesium oxide is 0.1 to 30 %(w/w), ethyl cellulose is 0.1 to 30 %(w/w), preferably, drug is 0.025 to 80 %(w/w), croscarmellose sodium is 2 to 25 %(w/w), potassium carbonate is 0.5 to 25 %(w/w), magnesium oxide is 0.5 to 25 %(w/w), ethyl cellulose is 0.5 to 25 %(w/w), more preferably, drug is 0.05 to 70 %(w/w), croscarmellose sodium is 3 to 20 %(w/w), potassium carbonate is 1 to 20 %(w/w), magnesium oxide is 1 to 20 %(w/w), ethyl cellulose is 1 to 20 %(w/w).

In a combination of drug/croscarmellose sodium/sodium carbonate/potassium carbonate/magnesium oxide/ethyl cellulose, each content relative to the amount of the whole formulation is, for example, drug is 0.01 to 90 %(w/w), croscarmellose sodium is 1 to 30 %(w/w), sodium carbonateand potassium carbonate is 0.1 to 30 %(w/w), magnesium oxide is 0.1 to 30 %(w/w), ethyl cellulose is 0.1 to 30 %(w/w), preferably, drug is 0.025 to 80 %(w/w), croscarmellose sodium is 2 to 25 %(w/w), sodium carbonateand potassium carbonate is 0.5 to 25 %(w/w), magnesium oxide is 0.5 to 25 %(w/w), ethyl cellulose is 0.5 to 25 %(w/w), more preferably, drug is 0.05 to 70 %(w/w), croscarmellose sodium is 3 to 20 %(w/w), sodium carbonate and potassium carbonate is 1 to 20 %(w/w), magnesium oxide is 1 to 20 %(w/w), ethyl cellulose is 1 to 20 %(w/w).

In a combination of drug/croscarmellose sodium/sodium carbonate/ethyl cellulose, each content relative to the amount of the whole formulation is, for example, drug is 0.01 to 90 %(w/w), croscarmellose sodium is 1 to 30 %(w/w), sodium carbonate is 0.1 to 30 %(w/w), ethyl cellulose is 0.1 to 30 %(w/w), preferably, drug is 0.025 to 80 %(w/w), croscarmellose sodium is 2 to 25 %(w/w), sodium carbonate is 0.5 to 25 %(w/w), ethyl cellulose is 0.5 to 25 %(w/w), more preferably, drug is 0.05 to 70 %(w/w), croscarmellose sodium is 3 to 20 %(w/w), sodium carbonate is 1 to 20 %(w/w), ethyl cellulose is 1 to 20 %(w/w).

In a combination of drug/croscarmellose sodium/potassium carbonate/ethyl cellulose, each content relative to the amount of the whole formulation is, for example, drug is 0.01 to 90 %(w/w), croscarmellose sodium is 1 to 30 %(w/w), potassium carbonate is 0.1 to 30 %(w/w), ethyl cellulose is 0.1 to 30 %(w/w), preferably, drug is 0.025 to 80 %(w/w), croscarmellose sodium is 2 to 25 %(w/w), magnesium oxide is 0.5 to 25 %(w/w), ethyl cellulose is 0.5 to 25 %(w/w), more preferably, drug is 0.05 to 70 %(w/w), croscarmellose sodium is 3 to 20 %(w/w), magnesium oxide is 1 to 20 %(w/w), ethyl cellulose is 1 to 20 %(w/w).

In a combination of drug/croscarmellose sodium/sodium carbonate/potassium carbonate/ethyl cellulose, each content relative to the amount of the whole formulation is, for example, drug is 0.01 to 90 %(w/w), croscarmellose sodium is 1 to 30 %(w/w), sodium carbonateand potassium carbonate is 0.1 to 30 %(w/w), ethyl cellulose is 0.1 to 30 %(w/w), preferably, drug is 0.025 to 80 %(w/w), croscarmellose sodium is 2 to 25 %(w/w), sodium carbonateand potassium carbonate is 0.5 to 25 %(w/w), ethyl cellulose is 0.5 to 25 %(w/w), more preferably, drug is 0.05 to 70 %(w/w), croscarmellose sodium is 3 to 20 %(w/w), sodium carbonate and potassium carbonate is 1 to 20 %(w/w), ethyl cellulose is 1 to 20 %(w/w).

The tablet of the present invention is useful as a sustained release formulation, and can control the dissolution rate compared to immediate release formulation which may not be control the elution. Especially, the dissolution rate can be controlled similarly with 1st fluid for dissolution test, 2nd fluid for dissolution test, distilled water or other buffer.

This formulation can be orally administered with water.

### EXAMPLES

The present invention is described in detail below by the following examples, comparative examples and reference examples, but they are not intended to limit the present invention. The tablet written in examples, comparative examples or reference examples was manufactured by following method. The dissolution rate of the drug was measured when the tablet eluted.

### (1) The effects of the alkali agent

To investigate the effects of the alkali agent, the tablet written in Table 1 was manufactured, and their dissolution rate was measured.

### a. Manufacturing method of the tablet

Prescription of tablet per one tablet is given in Table 1. Acetaminophen (Yamamoto Corporation) was used as a drug. Magnesium oxide (Kyowa Chemical Industry) was used as an alkali agent. Mannitol (Roquette), microcrystalline cellulose (Asahi Kasei Chemicals Corporation), croscarmellose sodium (FMC Corporation), sodium carbonate (Wako pure Chemical Industries), povidone (Nippon Shokubai) and magnesium stearate (Taihei Chemical Industrial) are used as other additive agents.

As the manufacturing method, acetaminophen, mannitol, microcrystalline cellulose, croscarmellose sodium, sodium carbonate, povidone and magnesium oxide are mixed, and water was added, and the granulated substance was manufactured by wet granulation.

The obtained granulated substance and magnesium stearate are mixed, and mix powder was obtained. This mix powder was tableted by single punch tablet machine.

A formulation of comparative example 1 was manufactured as immediate release formulation.

### b. Dissolution test

Dissolution test was carried out in accordance with the dissolution test written in the 16th Japanese Pharmacopoeia. 1st fluid for dissolution test (pH1.2), 2nd fluid for dissolution test (pH6.8) and distilled water were used as dissolution test solution.

### c. Disintegration test

Disintegration of the tablet was checked in accordance with the dissolution test solution written in the 16th Japanese Pharmacopoeia. Disintegration test was carried out by using the test solution (1st fluid for disintegration test of Japanese Pharmacopoeia) for 120 minutes, and disintegration of the tablet was checked.

**Table 1**

| (units w/w%) | | | |
|---|---|---|---|
| | comparative example 1 | comparative example 2 | example 1 |
| Acetaminophen | 50.00 | 50.00 | 50.00 |
| Mannitol | 28.50 | 21.70 | 11.70 |
| Microcrystalline cellulose | 12.25 | 9.30 | 9.30 |
| Croscarmellose sodium | 6.25 | 10.00 | 10.00 |
| Sodium carbonate | - | 6.00 | 6.00 |
| Magnesium oxide | - | - | 10.00 |
| Povidone | 2.00 | 2.00 | 2.00 |
| Magnesium stearate | 1.00 | 1.00 | 1.00 |
| SUM | 100.00 | 100.00 | 100.00 |

### d. Results

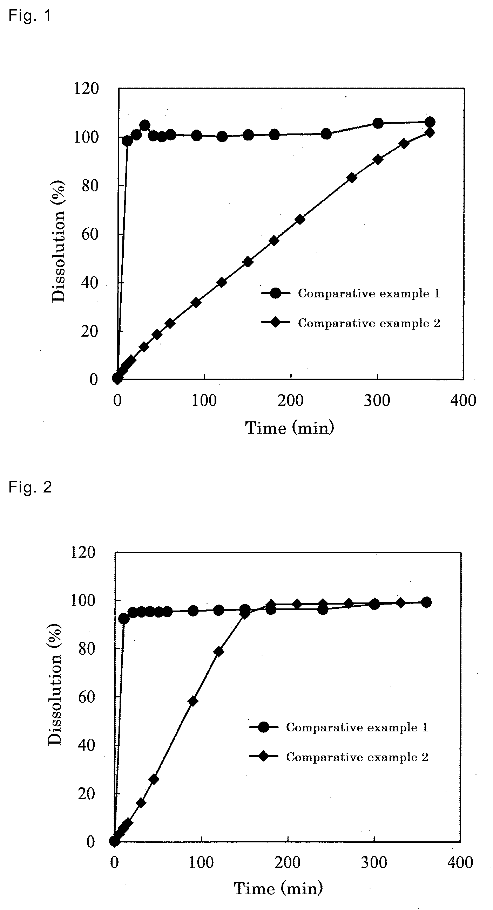

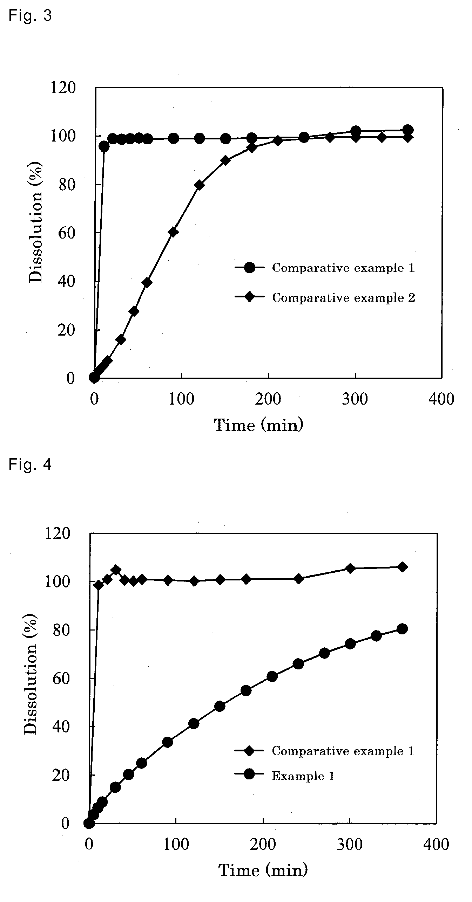

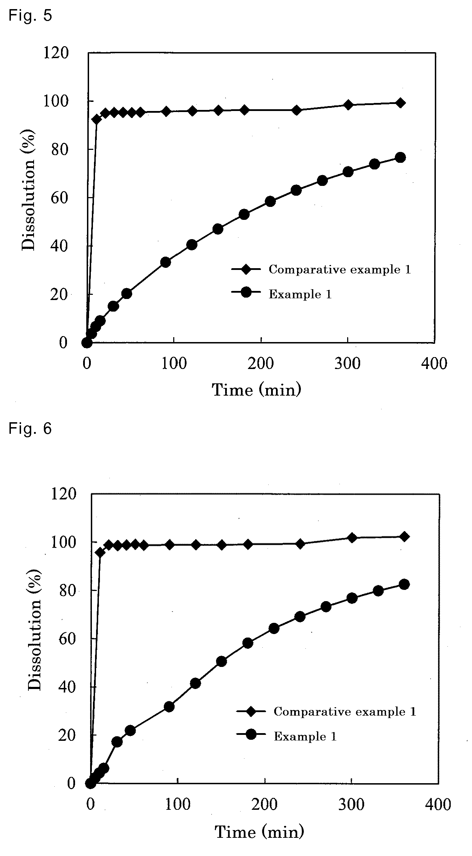

The results of dissolution test of comparative example 1, 2 and example 1 written in Table 1 are given in Figure 1 to 6 in accordance with pH difference of dissolution test solution. The formulation of comparative example 2 containing croscarmellose sodium and sodium carbonate controlled dissolution compared to the formulation of comparative example 1 which may not control dissolution. However, dissolution behavior of the formulation of comparative example 2 was different in various pH of dissolution test solution or distilled water (Figure 1 to 3). On the other hand, the dissolution behavior of the formulation of example 1 containing croscarmellose sodium, sodium carbonate and magnesium oxide was not very different in each dissolution test solution or distilled water (Figure 4 to 6). Accordingly, by adding croscarmellose sodium, sodium salt such as sodium carbonate, and alkali agent such as magnesium oxide, dissolution can be controlled with same dissolution behavior regardless of dissolution test solution. The formulation of comparative example 1 and comparative example 2 non-adding alkali agent disintegrated immediately in disintegration test, but the formulation of example 1 could be substantially delay disintegration time compared with the formulation of comparative example 1 and 2, even though the formulation of example 1 disintegrated within 120 minutes.

### (2) The dissolution behavior of the sustained release formulation containing the neutral drug (acetaminophen)

To look at the difference of dissolution behavior in drugs, the tablets written in Table 2 were manufactured and, their dissolution speed was measured.

### a. Manufacturing method of the tablet

Prescription of tablet per one tablet is given in Table 2. Acetaminophen (Yamamoto Corporation), a neutral drug, was used as a drug. Mannitol (Roquette), microcrystalline cellulose (Asahi Kasei Chemicals Corporation), croscarmellose sodium (FMC Corporation), magnesium oxide (Kyowa Chemical Industry) or magnesium hydroxide (Kyowa Chemical Industry), sodium carbonate (Wako pure Chemical Industries) or potassium carbonate (Wako pure Chemical Industries), hypromellose (Shin-Etsu Chemical), povidone (Nippon Shokubai) and magnesium stearate (Taihei Chemical Industrial) are used as other additive agents.

As the manufacturing method, drug, mannitol, microcrystalline cellulose, croscarmellose sodium, hypromellose, sodium carbonate or potassium carbonate, magnesium oxide or magnesium hydroxide are mixed, and water was added, and the granulated substance was manufactured by wet granulation in a similar manner to example 1.

The obtained granulated substance and magnesium stearate are mixed, and mix powder was obtained. This mix powder was tableted by single punch tablet machine.

### b. Dissolution test

Dissolution test was carried out in accordance with the dissolution test written in the 16th Japanese Pharmacopoeia. 2nd fluid for dissolution test (pH6.8) was used as dissolution test solution.

### c. Disintegration test

Disintegration of the tablet was checked in accordance with the dissolution test written in the 16th Japanese Pharmacopoeia. Disintegration test was carried out by using the test solution (1st fluid for disintegration test of Japanese Pharmacopoeia) for 120 minutes, and disintegration of the tablet was checked.

**Table 2**

| (units w/w%) | | | | | |
|---|---|---|---|---|---|
| | comparative example 1 | example 1 | example 2 | example 3 | example 4 |
| Acetaminophen | 50.00 | 50.00 | 50.00 | 50.00 | 50.00 |
| Mannitol | 28.50 | 11.70 | 6.70 | 6.70 | 6.70 |
| Microcrystalline cellulose | 12.25 | 9.30 | 9.30 | 9.30 | 9.30 |
| Croscarmellose sodium | 6.25 | 10.00 | 10.00 | 10.00 | 10.00 |
| Hypromellose | - | - | 10.00 | 10.00 | 10.00 |
| Sodium carbonate | - | 6.00 | 6.00 | 6.00 | - |
| Potassium carbonate | - | - | - | - | 6.00 |
| Magnesium oxide | - | 10.00 | 5.00 | - | 5.00 |
| Magnesium hydroxide | - | - | - | 5.00 | - |
| Povidone | 2.00 | 2.00 | 2.00 | 2.00 | 2.00 |
| Magnesium stearate | 1.00 | 1.00 | 1.00 | 1.00 | 1.00 |
| SUM | 100.00 | 100.00 | 100.00 | 100.00 | 100.00 |

### d. Results

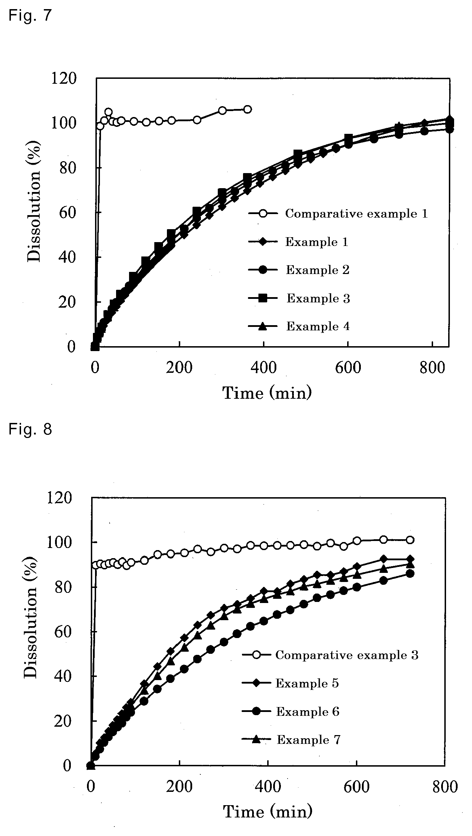

The results of dissolution test of comparative example 1 and examples 1 to 4 written in Table 2 are given in Figure 7. The formulations of example 1 and 2 could be swelled and control dissolution compared to the formulation of comparative example 1. Moreover, the formulation of example 1 disintegrated within 120 minutes in disintegration test, but the formulation of example 2 sustained the form almost. It appeared that the formulation could be sustaining by peristaltic movement of the gastrointestinal tract. As the formulations of example 3 and example 4 were similar behavior to the formulation of example 2, similar dissolution control effects had been confirmed, in the case that potassium salt as substitute for sodium salt, and magnesium hydroxide as substitute for magnesium oxide were used.

### (3) The dissolution behavior of the sustained release formulation containing the acid drug (ibuprofen)

### a. Manufacturing method of the tablet

Formulation of tablet per one tablet is given in Table 3. Ibuprofen (Hachidai Pharmaceutical), an acid drug, was used as a drug, and ethyl cellulose was used as hydrophobic base. The other additive agents and the manufacture method are similar manner to example 2.

### b. Dissolution test

Dissolution test was carried out in accordance with the dissolution test written in the 16th Japanese Pharmacopoeia. 2nd fluid for dissolution test (pH6.8) was used as dissolution test solution.

### c. Disintegration test

Disintegration of the tablet was checked in accordance with the dissolution test written in the 16th Japanese Pharmacopoeia. Disintegration test was carried out by using the test solution (1st fluid for disintegration test of Japanese Pharmacopoeia) for 120 minutes, and disintegration of the tablet was checked.

**Table 3**

| (units w/w%) | | | | |
|---|---|---|---|---|
| | comparative example 3 | example 5 | example 6 | example 7 |
| Ibuprofen | 50.00 | 50.00 | 50.00 | 50.00 |
| Mannitol | 27.70 | 11.70 | 6.20 | 6.13 |
| Microcrystalline cellulose | 9.30 | 9.30 | 9.30 | 4.87 |
| Croscarmellose sodium | 10.00 | 10.00 | 10.00 | 10.00 |
| Hypromellose | - | - | 10.00 | - |
| Ethyl cellulose | - | - | - | 10.00 |
| Sodium carbonate | - | 6.00 | 6.00 | 6.00 |
| Magnesium oxide | - | 10.00 | 5.50 | 10.00 |
| Povidone | 2.00 | 2.00 | 2.00 | 2.00 |
| Magnesium stearate | 1.00 | 1.00 | 1.00 | 1.00 |
| SUM | 100.00 | 100.00 | 100.00 | 100.00 |

### d. Results

The results of dissolution test of comparative example 3 and examples 5 to 7 written in Table 3 are given in Figure 8. The formulations of examples 5 to 7 could control dissolution compared to the formulation of comparative example 3. The formulation of example 5 in 2nd fluid for dissolution test could be swelled and controlled dissolution, but the formulation was disintegrated in the disintegration test. On the other hand, the formulations of example 6 and 7 were swelled in 2nd fluid for dissolution test and could control dissolution. In addition, as these formulations weren't disintegrated in the disintegration test, it appeared that these formulations could be sustaining by peristaltic movement of the gastrointestinal tract.

### (4) The dissolution behavior of the sustained release formulation containing the basic drug (nicardipine hydrochloride)

### a. Manufacturing method of the tablet

Formulation of tablet per one tablet is given in Table 4. Nicardipine (Tokyo Chemical Industry), a basic drug, was used as a drug. The other additive agents and the manufacture method are similar manner to example 2.

### b. Dissolution test

Dissolution test was carried out in accordance with the dissolution test written in the 16th Japanese Pharmacopoeia. 2nd fluid for dissolution test (pH6.8) containing 0.2% Tween20 was used as dissolution test solution.

### c. Disintegration test

Disintegration of the tablet was validated in accordance with the dissolution test written in the 16th Japanese Pharmacopoeia. Disintegration test was carried out by using the test solution (1st fluid for disintegration test of Japanese Pharmacopoeia) for 120 minutes, and disintegration of the tablet was validated.

**Table 4**

| (units w/w%) | | | | | |
|---|---|---|---|---|---|
| | comparative example 4 | example 8 | example 9 | example 10 | example 11 |
| Nicardipine hydrochloride, | 50.00 | 50.00 | 50.00 | 50.00 | 50.00 |
| Mannitol | 27.70 | 6.70 | 14.50 | 13.10 | 11.70 |
| Microcrystalline cellulose | 9.30 | 9.30 | 11.50 | 10.40 | 9.30 |
| Croscarmellose sodium | 10.00 | 10.00 | 10.00 | 10.00 | 10.00 |
| Hypromellose | - | 10.00 | 5.00 | 7.50 | 10.00 |
| Sodium carbonate | - | 6.00 | 6.00 | 6.00 | 6.00 |
| Magnesium oxide | - | 5.00 | - | - | - |
| Povidone | 2.00 | 2.00 | 2.00 | 2.00 | 2.00 |
| Magnesium stearate | 1.00 | 1.00 | 1.00 | 1.00 | 1.00 |
| SUM | 100.00 | 100.00 | 100.00 | 100.00 | 100.00 |

### d. Results

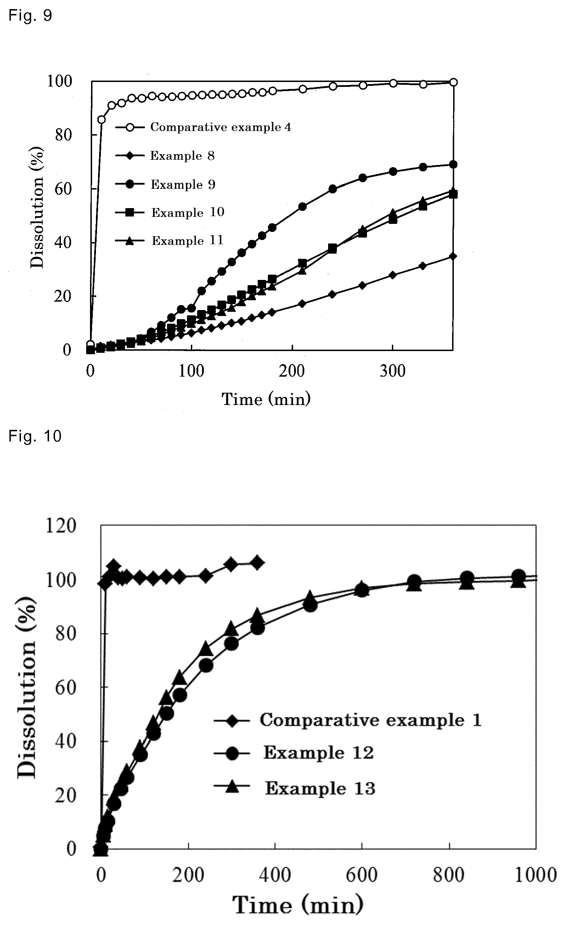

The results of dissolution test of comparative example 4 and examples 8 to 11 written in Table 4 are given in Figure 9. The formulations of example 8 could control dissolution compared to the formulation of comparative example 4. The formulation of examples 8 to 11 in 2nd fluid for dissolution test could be swelled and controlled dissolution. Moreover, it appeared that these formulations could be sustaining by peristaltic movement of the gastrointestinal tract as these formulations weren't disintegrated in the disintegration test. The formulations of examples 9 to 11 sustained the sufficient intensity of the tablet without alkali agent. Dissolution rate decreased in the formulations of example 9 to 11 when quantity of hypromellose was increased.

### (5) The state of formulation after animal experiment

### a. Experimental method

Formulation of tablet per one tablet is given in Table 5. Compound A, an acid drug, was used as a drug. The dogs were dissected after 2 hours and 6 hours after administrating the present formulation, and the state of the formulation was observed.

**Table 5**

| | (units w/w%) |
|---|---|
| | formulation for animal experiment |
| Compound A | 50.00 |
| Mannitol | 14.70 |
| Microcrystalline cellulose | 9.30 |
| Croscarmellose sodium | 5.00 |
| Hypromellose | 10.00 |
| Sodium carbonate | 3.00 |
| Magnesium oxide | 5.00 |
| Povidone | 2.00 |
| Magnesium stearate | 1.00 |
| SUM | 100.00 |

### b. Results

The administrated dogs were dissected, and tablets were picked up. The remaining ratio was about 70% in 2 hours later and about 22% in 6 hours later. These formulations found in the vicinity of pylorus, and had much intensity after administration. Accordingly, the formulation, which eluted a drug gradually and could be sustaining by peristaltic movement of the gastrointestinal tract, could be manufactured.

### (6) The effects of the oleaginous base

To look at the effects of the oleaginous base, the tablet written in Table 6 was manufactured, and their dissolution rate was measured.

### a. Manufacturing method of the tablet

Formulation of tablet per one tablet is given in Table 1. Acetaminophen (Yamamoto Corporation) was used as a drug. Magnesium oxide (Kyowa Chemical Industry) was used as an alkali agent. Sucrose fatty acid ester (Mitsubishi-Kagaku Foods Corporation) or hardened oil (Freund Corporation) was used as a oleaginous base. The other additive agents and the manufacture method are similar manner to example 1.

### b. Dissolution test

Dissolution test was carried out in accordance with the dissolution test written in the 16th Japanese Pharmacopoeia. 2nd fluid for dissolution test (pH6.8) was used as dissolution test solution.

### c. Disintegration test

Disintegration of the tablet was validated in accordance with the dissolution test written in the 16th Japanese Pharmacopoeia. Disintegration test was carried out by using the test solution (1st fluid for disintegration test of Japanese Pharmacopoeia) for 120 minutes, and disintegration of the tablet was validated.

**Table 6**

| (units w/w%) | | | |
|---|---|---|---|
| | comparative example 1 | example 12 | example 13 |
| Acetaminophen | 50.00 | 50.00 | 50.00 |
| Mannitol | 28.50 | 14.60 | 14.60 |
| Microcrystalline cellulose | 12.25 | 9.40 | 9.40 |
| Croscarmellose sodium | 6.25 | 5.00 | 5.00 |
| Sodium carbonate | - | 3.00 | 3.00 |
| Sucrose fatty acid ester | - | 10.00 | - |
| Hardened oil | - | - | 10.00 |
| Magnesium oxide | - | 5.00 | 5.00 |
| Povidone | 2.00 | 2.00 | 2.00 |
| Magnesium stearate | 1.00 | 1.00 | 1.00 |
| SUM | 100.00 | 100.00 | 100.00 |

### d. Results

The results of dissolution test of comparative example 1 and examples 12 and 13 written in Table 6 are given in Figure 10. As the formulations of example 12 and 13 could be swelled and control dissolution compared to the formulation of comparative example 1, disintegration time was delayed substantially. This means that the formulations of example 12 and 13 are sustainable to peristaltic movement of the gastrointestinal tract. This result shows that similar dissolution control effects had been confirmed in the case that sucrose fatty acid ester or hardened oil as an oleaginous base was used.

### INDUSTRIAL APPLICABILITY

The sustained release formulation of the present invention is able to control dissolution rate and sustain by peristaltic movement of the gastrointestinal tract. Hence, it is useful for a sustained release formulation of a drug whose site of absorption is small intestine or large intestine.

## Claims

1. A solid dosage form comprising a drug, croscarmellose sodium, an alkali metal salt or an alkoxide, and an alkali agent, wherein the alkali metal salt is a sodium salt or a potassium salt, and the alkali agent is a magnesium oxide.

2. The solid dosage form according to claim 1, which comprises the alkali metal salt, wherein the alkali metal salt is one or more selected from the group consisting of sodium carbonate, sodium hydrogen carbonate and potassium carbonate.

3. The solid dosage form according to claim 2, wherein the alkali metal salt is sodium carbonate.

4. A solid dosage form comprising a drug, croscarmellose sodium, an alkali metal salt or an alkoxide, and a water soluble polymer or a hydrophobic base, wherein the alkali metal salt is a sodium salt or a potassium salt.

5. The solid dosage form according to claim 4, wherein the drug is a basic drug.

6. The solid dosage form according to claim 4 or 5, which further comprises an alkali agent.

7. The solid dosage form according to any one of claims 4 to 6, which comprises an alkali metal salt, wherein the alkali metal salt is one or more selected from the group consisting of sodium carbonate, sodium hydrogen carbonate and potassium carbonate.

8. The solid dosage form according to claim 6, wherein the alkali agent is one or more selected from the group consisting of magnesium oxide, magnesium hydroxide, aluminum magnesium hydroxide, synthetic hydrotalcite, calcium carbonate, magnesium carbonate, magnesium aluminometa silicate and calcium silicate.

9. The solid dosage form according to claim 8, wherein the alkali agent is magnesium oxide.

10. The solid dosage form according to any one of claims 4 to 9, which comprises a hydrophobic base, wherein the hydrophobic base is a water-soluble polymer or oleaginous base.

11. The solid dosage form according to claim 10, wherein the hydrophobic base is a water-soluble polymer, wherein the water-soluble polymer is one or more selected from the group consisting of cellulose-based polymer, vinyl-based polymer and acrylic-based polymer.

12. The solid dosage form according to claim 11, wherein the water-soluble polymer is one or more selected from the group consisting of ethyl cellulose, low substituted hydroxypropyl cellulose, aminoalkylmetaacrylatecopolymer, polyethylacrylate-methylmethacrylate-trimethylammmonioethylmethacrylatechlorid, cellulose acetate phthalate, methacrylate copolymer, hydroxypropylmethylcellulose phthalate, hydroxypropylmethylcellulose acetate succinate, carboxymethylethyl cellulose and carboxyvinyl polymer.

13. The solid dosage form according to claim 10, wherein the hydrophobic base is oleaginous base, wherein the oleaginous base is one or more selected from the group consisting of paraffin, microcrystalline wax, ceresin, vegetable wax, cacao butter, carnauba wax, beeswax, cetanol, stearyl alcohol, myristic acid, palmitic acid, stearic acid, glycerol fatty acid ester, polyglycerol fatty acid ester, glycerol free fatty acid ester, propylene glycol fatty acid ester, sorbitan fatty acid ester, sucrose fatty acid ester and hardened oil.

14. The solid dosage form according to any one of claims 4 to 9, which comprises a water-soluble polymer, wherein the water-soluble polymer is one or more selected from the group consisting of cellulose-based polymer, vinyl-based polymer and acrylic-based polymer.

15. The solid dosage form according to claim 14, wherein the water-soluble polymer is one or more selected from the group consisting of hypromellose, crospovidone, povidone, sodium polyacrylate, sodium alginate and polyethylene oxide.

16. The solid dosage form according to claim 6, which comprises an alkali metal salt, a water soluble polymer and an alkali agent, wherein the water soluble polymer is hypromellose and the alkali agent is magnesium oxide.

17. The solid dosage form according to any one of claims 1 to 16, wherein the content of the drug in the formulation is 0.01 to 90 %(w/w).

18. The solid dosage form according to any one of claims 1 to 3 and 6 to 17, wherein the content of the alkali agent in the formulation is 0.1 to 30 %(w/w).

19. The solid dosage form according to claim 15 or 16, wherein the content of hypromellose in the formulation is 0.1 to 30 %(w/w).

20. The solid dosage form according to claim 3, wherein the content of the drug in the formulation is 0.01 to 90 %(w/w), and the content of magnesium oxide in the formulation is 0.1 to 30 %(w/w).

21. The solid dosage form according to claim 16, wherein the content of the drug in the formulation is 0.01 to 90 %(w/w), the content of magnesium oxide in the formulation is 0.1 to 30 %(w/w), and the content of hypromellose in the formulation is 0.1 to 30 % (w/w).

22. The solid dosage form according to any one of claims 1 to 21, wherein the solid dosage form is a tablet or a granule.
